# EUROPEAN PATENT SPECIFICATION

(11) **EP 2 490 676 B1**
(45) Date of publication and mention of the grant of the patent: **11.12.2019**
(21) Application number: 10782693.5
(22) Date of filing: 20.10.2010
(51) Int. Cl.: A61K 9/24, A61K 31/4985, A61K 31/138

(54) **PHARMACEUTICAL COMPOSITIONS OF PDE-5 INHIBITORS AND DAPOXETINE**
PHARMAZEUTISCHE ZUSAMMENSETZUNG EINES PDE-5 INHIBITORS UND DAPOXETIN
LA COMPOSITION PHARMACEUTIQUE DES INHIBITEURS DE LA PDE-5 ET DAPOXETINE

(30) Priority: 22.10.2009 EP 09173724
(43) Date of publication of application: 29.08.2012
(73) Proprietor: Abdi Ibrahim Ilac Sanayi ve Ticaret Anonim Sirketi, Istanbul (TR)
(72) Inventor: FARSHI, Farhad, 34555 Istanbul (TR); KOC, Fikret, 34555 Istanbul (TR); SOYLEMEZ, Serdar, 34555 Istanbul (TR); KANDEMIR, Levent, 34555 Istanbul (TR)
(86) International application number: PCT/IB2010/054740
(87) International publication number: WO 2011/048553

(56) References cited:
- EP-A2- 2 591 773
- WO-A1-2007/002125
- WO-A2-2007/000764
- WO-A2-2008/146178
- IN-A1- 1128M UM2 008
- US-B1- 6 403 597
- LU SHAOMING ET AL: "Combined use of phosphodiesterase-5 inhibitors and selective serotonin reuptake inhibitors for temporary ejaculation failure in couple undergoing assisted reproductive technologies.", FERTILITY AND STERILITY MAY 2009, vol. 91, no. 5, May 2009 (2009-05), pages 1806-1808, XP002570491, ISSN: 1556-5653
- DRESSER M J ET AL: "Dapoxetine, a novel treatment for premature ejaculation, does not have pharmacokinetic interactions with phosphodiesterase-5 inhibitors.", INTERNATIONAL JOURNAL OF IMPOTENCE RESEARCH 2006 JAN-FEB, vol. 18, no. 1, January 2006 (2006-01), pages 104-110, XP9130077, ISSN: 0955-9930 cited in the application
- Prigily 30 mg and 60 mg film- coated tablets, https://www.medicines.org.uk/emc/
- Register excerpt from http://ipindiaonline.gov.in/patentsearch/s earch/index.aspx
- CPMP/EWP/280/96 Carr*, Note for Guidance on Modified Release Oral and Transdermal Dosage Forms", EMA's Committee for Proprietary Medicinal Products (CPMP) on July 28th, 1999
- Wikipedia Excerpt (Dapoxetine)
- ANDERSSON KARL-ERIK ET AL: "Pharmacokinetic and pharmacodynamic features of dapoxetine, a novel drug for 'on-demand' treatment of premature ejaculation", BJU INTERNATIONAL, vol. 97, no. 2, February 2006 (2006-02), pages 311-315, ISSN: 1464-4096
- Decision T367/11

## Description

### Technical Field

This invention is related to pharmaceutical formulations of selective type 5 PDE-5 inhibitors and selective serotonin reuptake inhibitor **(SSRI)** which is used in the treatment of premature ejaculation, such as dapoxetine, combination. This invention is also directed to preparation methods for said combination.

### Background Art

PCT/WO 03000343 (VIVUS, INC., 21.06.2001) discloses phosphodiesterase inhibitor and dapoxetine combination is administered transmucosally, e.g., via the sublingual, buccal, nasal, or rectal routes, or via inhalation.

Dapoxetine and PDE-5 inhibitors combination is known in the prior art. For example; DRESSER, et al. 'Dapoxetine, a novel treatment for premature ejaculation, does not have pharmacokinetic interactions with phosphodiestefase-5 inhibitors'. Int. J. Import Res.. 2006, vol.18, no.1.

EP2591773 A2 (NAVIPHARM CO LTD) discloses time-delayed sustained release pharmaceutical composition comprising Dapoxetine, which is comprised of an immediate release phase dapoxetine and a prologed sustained release phase and said immediate release phase and prolonged sustained release respectively contain 20-80% of the entire Dapoxetine contents.

IN /1128/MUM/2008 describes a pharmaceutical composition comprising a combination of Dapoxetine and phosphodiesterase V inhibitor and gives general information about formulation of this combination.

### Disclosure of Invention

Selective type 5 cGMP phosphodiesterase (PDE-5) inhibitors are used in treatment of erectile dysfunction and have a elimination half life of at least 4-5 hours in the case of tadalafil the elimination half life is about 17 hours.

Dapoxetine hydrochloride (Priligy®) is a short-acting SSRI drug for the treatment of premature ejaculation in men and has an elimination half-life of approximately 1.5 h.

This invention is related to pharmaceutical compositions of a PDE-5 inhibitor or mixtures of PDE-5 inhibitors of pharmaceutically acceptable salts thereof and a selective serotonin reuptake inhibitor **(SSRI)** or mixtures of selective serotonin reuptake inhibitors of pharmaceutically acceptable salts thereof wherein the selective serotonin reuptake inhibitors are used in the treatment of premature ejaculation.

Another issue of this invention is related to close the big difference in elimination half-life between dapoxetine and PDE-5 inhibitors by using different sustained of modified release but not limited to these formulations.

These formulations aim for modification of the release profile to have efficient dapoxetine blood level concentration for a longer period of time than without modification.

According to this invention the term 'phosphodiesterase inhibitor' or 'PDE 5 inhibitor' includes phosphodiesterase inhibitors per se as well as pharmaceutically acceptable and pharmacologically active salts, esters, amides, prodrugs, active metabolites, other derivatives, mixtures thereof and the like.

The term 'therapeutically effective amount' is meant a nontoxic but sufficient amount of the drug of agent to provide the desired effect.

According to this invention (SSRI) includes selective serotonin reuptake inhibitors per se as well as pharmaceutically acceptable and pharmacologically active salts, esters, amides, prodrugs, active metabolites, other derivatives, mixtures thereof and the like.

PDE-5 selective inhibitors are, but not limited to, sildenafil, tadalafil, vardenafil, udenafil, avanafil, dasantafil, SLx2101 and LAS34179, mixtures thefeof and the tike. Pfeferred PDE5 inhibitof is tadalafil.

Serotonin reuptake inhibitors (SSRI), which used in the treatment of premature ejaculation, are but not limited to fluoxetine, dapoxetine, sertraline, citalopram, escitalopram, venlafaxine, mixtures thereof and the like. Preferred serotonin reuptake inhibitor is dapoxetine and especially its hydrochloride salt.

According to this invention pharmaceutical compositions may be in the form of tablet, bilayer tablet, tablet in tablet, capsule, tablet in capsule, pellets in capsule, granules in capsule and other dosage forms suitable for oral administration.

In one embodiment, this invention embraces bilayer pharmaceutical tablet. Bilayer pharmaceutical tablet is comprising; a) a first layer containing a PDE 5 inhibitor or mixtures of PDE 5 inhibitors or pharmaceutically acceptable salts thereof and a pharmaceutically acceptable carrier or carries and b) a second layer containing dapoxetine or pharmaceutically acceptable salts thereof and a pharmaceutically acceptable carrier or carries.

Pharmaceutical formulations of combination can be prepared by one of the method as following manners; a) Active pharmaceutical ingredients are granulated separately and then granulates are assembled namely the active pharmaceutical ingredients are each dispersed within its own pharmaceutically acceptable carrier or carries. One of them may be direct compression (DC) form b) one active pharmaceutical ingredient is granulated and then granulated active pharmaceutical ingredient is assembled with non-granulated active pharmaceutical ingredient c) Both active pharmaceutical ingredients are granulated together d) One active pharmaceuticals ingredient is dissolved alone or with one or more excipient and then other active pharmaceutical ingredient alone or with one or more excipients are coated with said solution. e) One active pharmaceutical ingredient is dispersed alone or with one or more excipients and then other active pharmaceutical ingredient alone or with one or more excipients are coated with said dispersion.

In the course of the preparation, direct compression, wet granulation, dry granulation methods or mixtures thereof can be applied.

Modified release formulations can be preferred. Modified release formulations are, but not limited to, controlled release, sustained release, delayed release, extended release, repeat action system, mixtures thereof and the like.

Pharmaceutical formulations can be made in two parts wherein one part is modified release formulation of PDE 5 inhibitor and another one is immediate release formulation of selective serotonin reuptake inhibitor (SSRI) or vice versa.

In other embodiment, this invention embraces bilayer pharmaceutical tablet. Bilayer pharmaceutical tablet is comprising; a) a first layer containing a PDE 5 inhibitor or mixtures of PDE 5 inhibitors or pharmaceutically acceptable salts thereof and a pharmaceutically acceptable carrier or carriers and b) a second layer containing dapoxetine or pharmaceutically acceptable salts thereof and a pharmaceutically acceptable carrier or carriers to provide dapoxetine in a release profile to have efficient dapoxetine blood level concentration fora longer period of time than without modification.

In another embodiment, this invention embraces bilayer pharmaceutical tablet. Bilayer pharmaceutical tablet is comprising; a) first layer containing an effective amount of dapoxetine or pharmaceutically acceptable salts thereof and a pharmaceutically acceptable carrier or carriers to provide dapoxetine in release profile to have efficient dapoxetine blood level concentration for a longer period of time than without modification when compared with separate formulation of dapoxetine which is launched under Priligy® trademark. b) a second layer containing a PDE 5 inhibitor or mixtures of PDE 5 inhibitors or pharmaceutically acceptable salts thereof anda initial amount of dapoxetine to provide an efficient amount of dapoxetine for initial effectivity and a pharmaceutically acceptable carrier or carriers.

The expression bilayer is not limited to two layers also multilayer tablets are meant with this expression.

PDE-5 inhibitors and selective serotonin reuptake inhibitor (SSRI) combination can be administered in therapeutically effective amounts.

According to this invention, combination includes one or more pharmaceutically acceptable excipients, carriers, or diluents. In formulations, surfactants, diluents, sweeteners, disintegrants, binders, lubricants, glidants, colorants, flavors and mixtures thereof can be used.

Diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharrnaburst™, mixtures thereof and the like.

Glidants are, but not limited to, silicon dioxide, colloidal silicon dioxide, calcium silicate, magnesium silicate, magnesium trisilicate, talc, starch, mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes, polyacrylamide, mixtures thereof, and the like.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like.

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose, lower alkylsubstituted hydroxypropyl cellulose, polacrilin potassium, starch, pregelatinized starch, sodium alginate, mixtures thereof or whatsoever.

This invention embraces pharmaceutical composition of therapeutically effective amount of PDE 5 inhibitor or mixtures of PDE 5 inhibitors or pharmaceutically acceptable salts thereof and therapeutically effective amount of selective serotonin reuptake inhibitor or mixtures of selective serotonin reuptake inhibitors or pharmaceutically acceptable salts thereof and use of said composition for the manufacture of a medicament for the treatment of disease or diseases where premature ejaculation is associated with erectile dysfunction.

### Example 1

**[Table 1]**

| Ingredients | Weight (mg) |
|---|---|
| Tadalafil | 20 |
| Dapoxetine | 60 |
| Lactose Monohydrate | 160 |
| Lactose Monohydrate (spray dried) | 30 |
| Hydroxypropy lcellulose | 6 |
| Sodium Lauryl Sulfate | 3 |
| Microcrystalline Cellulose | 45 |
| Magnesium Stearate | 3 |
| Colloidal Silicon Dioxide | 7 |
| Croscarmellose Sodium | 10 |

## Claims

1. The bilayer tablet comprising; a) a first layer containing a PDE 5 inhibitor or mixtures of PDE 5 inhibitors or their pharmaceutically acceptable salts thereof and a pharmaceutically acceptable carrier or carriers and b) a second layer containing dapoxetine or its pharmaceutically acceptable salt thereof and a pharmaceutically acceptable carrier or carriers wherein the first layer is an immediate release formulation and the second layer is a modified release formulation to provide dapoxetine in a release profile to have efficient dapoxetine blood level concentration for a longer period of time than without modification.

2. The bilayer tablet according to Claim 1 wherein; the first layer containing a PDE 5 inhibitor or mixtures of PDE 5 inhibitors or their pharmaceutically acceptable salts thereof also includes an initial amount of dapoxetine.

3. The bilayer tablet according to any of the preceding claims; wherein the PDE 5 inhibitor is tadalafil.

4. The bilayer composition according to any of the preceding claims further containing excipients comprising at least one or a mixture of diluents, glidants, binders, lubricants, disintegrants.

## Patentansprüche

1. Zweischichttablette umfassend a) eine erste Schicht ethältend einen PDE5-Inhibitor oder Mischungen von PDE5-Inhibitoren oder pharmazeutisch verträgliche Salzen davon und einen pharmazeutisch verträglichen Träger oder pharmazeutisch verträgliche Trägern und b) eine zweite Schicht ethältend Dapoxetin oder ein pharmazeutisch verträgliches Salz davon und einen pharmazeutisch verträglichen Träger oder pharmazeutisch verträgliche Trägern worin die erste Schicht eine Formulation mit sofortiger Freisetzung ist und die zweite Schicht eine Formulation mit modifizerter Freisetzung ist, um Dapoxetin in so einem Freisetzungsprofil bereitzustellen, dass eine wirksame Blutspiegelkonzentration von Dapoxetin für einen längeren Zeitraum als ohne Modifizerung sichergestellt wird.

2. Zweischichttablette gemäß Anspruch 1 wobei die erste Schicht ethältend einen PDE5-Inhibitor oder Mischungen von PDE5-Inhibitoren oder pharmazeutisch verträgliche Salzen davon auch eine Ausgangsmenge von Dapoxetin umfasst.

3. Zweischichttablette nach einem den vorhergehenden Ansprüchen worin der PDE5-Inhibitor Tadalafil ist.

4. Zweischichtzusammensetzung nach einem der vorhergehenden Ansprüchen ethältend weiterhin Hilfsstoffe umfassend mindestens ein Verdünnungsmittel, ein Fließregulierungsmittel, ein Bindemittel, ein Schmiermittel, ein Sprengmittel oder eine Mischung davon.

## Revendications

1. Le comprimé bicouche comprenant : a) une première couche contenant un inhibiteur PDE 5 ou mélanges des inhibiteurs PDE 5 ou les sels pharmaceutiquement acceptables de ceux-ci et un véhicule pharmaceutiquement acceptable ou des véhicules pharmaceutiquement acceptables et b) une seconde couche contenant la dapoxetine ou le sel pharmaceutiquement acceptable de celle-ci et un véhicule pharmaceutiquement acceptable ou des véhicules pharmaceutiquement acceptables, dans lequel la première couche est une formulation à libération immédiate et la seconde couche est une formulation à libération modifiée pour fournir la dapoxetine sous un profil de libération qui va permettre avoir une concentration sanguine efficace pendent une période plus longue que sans modification.

2. Le comprimé bicouche selon la revendication 1, dans lequel la première couche contenant un inhibiteur PDE 5 ou mélanges des inhibiteurs PDE 5 ou les sels pharmaceutiquement acceptables de ceux-ci, inclut aussi une quantité initiale de la dapoxetine.

3. Le comprimé bicouche selon l'une quelconque des revendications précédentes, dans lequel le inhibiteur PDE 5 est le tadalafil.

4. La composition bicouche selon l'une quelconque des revendications précédentes contenant, de plus, les excipients comprenant au moins un ou un mélange de diluants, agents de glissement, liants, lubrifiants et désintégrants.
